# EUROPEAN PATENT SPECIFICATION

(11) **EP 0 991 765 B1**
(45) Date of publication and mention of the grant of the patent: **08.12.2004**
(21) Application number: 98917476.8
(22) Date of filing: 24.04.1998
(51) Int. Cl.: C12N 15/54, C12N 15/82, C12N 5/10, A01H 5/00

(54) **RESISTANCE MARKER FOR MONOCOTYLEDONS**
RESISTENZMARKER FÜR EINKEIMBLÄTTRIGE PFLANZEN
MARQUEUR DE RESISTANCE POUR MONOCOTYLEDONES

(30) Priority: 25.04.1997 GB 9708542
(43) Date of publication of application: 12.04.2000
(73) Proprietor: BIOGEMMA UK LIMITED, Cambridge CB4 4GZ (GB)
(72) Inventor: FREEMAN, Judy, Patricia, Biogemma UK Limited, Cambridge CB4 4GZ (GB); BOWDEN, Sarah, Louise, Biogemma UK Limited, Cambridge CB4 4GZ (GB)
(74) Representative: Flesselles, Bruno F.G.
(86) International application number: PCT/GB1998/001221
(87) International publication number: WO 1998/049316

(56) References cited:
- EP-A- 0 369 637
- EP-A- 0 730 030
- WO-A-91/10725
- LI Z. ET AL.: "A sulfonylurea herbicide resistance gene from Arabidopsis thaliana as a new selectable marker for production of fertile transgenic rice plants" PLANT PHYSIOLOGY, vol. 100, no. 2, October 1992, pages 662-668, XP002076805
- WEEKS J T ET AL: "RAPID PRODUCTION OF MULTIPLE INDEPENDENT LINES OF FERTILE TRANSGENIC WHEAT (TRITICUM AESTIVUM)" PLANT PHYSIOLOGY, vol. 102, 1 January 1993, pages 1077-1084, XP000590886 cited in the application

## Description

The present invention relates to the use of sulphonamide resistance genes as selectable markers in wheat.

The use of recombinant DNA technology in the field of agriculture is becoming increasingly important. However, the application of such technology to monocot species, such as wheat, maize, rice and barley, was initially hampered by a lack of suitable transformation techniques. Gordon-Kamm *et al (Plant Cell*, **2**:603-618 (1990)) reported stable transformation of maize using microprojectile-mediated DNA delivery. Subsequently, Vasil *et al (Bio-technology,* **10:**667-674 (1992)) reported transformation of wheat following microprojectile bombardment of embryogenic callus. The use of immature embryos as the target tissue provided improved transformation methods for wheat (Weeks *et al*, *Plant Physiol.,* **102**:1077-1084 (1993); EP-A-0709462 (Monsanto)).

The *nptll* gene, conferring resistance to the antibiotic kanamycin, is the most widely used selectable marker for the transformation of dicotyledonous plant species. Kanamycin is unsuitable as a selective agent for monocot transformation however, due to high levels of tolerance to the antibiotic in these species. In addition, concerns have been raised in certain quarters regarding the use of such markers, and the consequent use of antibiotics for selection purposes, in the agricultural field. Whilst such concerns may be somewhat misplaced, the fact remains that, commercially, it is desirable to have available markers which are not antibiotic-based. Consequently, it has been necessary to look elsewhere for a marker gene suitable for use in monocot transformation systems (Wilmink & Dons *Plant Molecular Biology Reporter* **11(2):**165-185 (1993)).

The *bar* gene from *Streptomyces hygroscopius,* conferring resistance to the phosphinothricin (PPT)-based herbicides bialaphos and Basta, has been used successfully as the selectable marker for maize transformation (Gordon-Kamm *et al,* (1990), *supra*) and consequently, has been the first choice as a selectable marker for the development of a wheat transformation system (Vasil *et al, Bio-technology*, **11:**1153-1158 (1993); Nehra *et al, The Plant Journal,* **5(2):**285-297 (1994); and Becker *et al, The Plant Journal*, **5(2):**299-307 (1994)). However, there are problems inherent in the use of the *bar* gene as a selectable marker in wheat. In particular, the selection is inefficient, resulting in a high frequency of untransformed shoots regenerating on selection and requiring a further screen (e.g. by PCR analysis or enzyme assay) of regenerated shoots to identify those carrying the marker gene.

A number of factors may be responsible for the difficulties encountered in applying PPT-based selection to monocot transformation, particularly wheat transformation. PPT acts by inhibiting glutamine synthetase, an enzyme which has a key regulatory role in nitrogen metabolism. Dekeyser *et al (Plant Physiol*., **90:**217-223 (1989)) reported that amino acids in the culture medium allowed growth of untransformed rice calli in the presence of PPT. They found that glutamine and other amino acids must be omitted from the culture medium for PPT-based selection to be effective. However, these media components may be required for successful shoot regeneration.

Furthermore, the *bar* gene confers resistance to PPT-based herbicides by encoding an enzyme, phosphinothricin acetyl transferase (PAT), which detoxifies the PPT by acetylation. A problem with selection systems based on this type of resistance mechanism is that untransformed tissue may be protected by surrounding transformed cells (Wilmink & Dons, (1993) *supra).* By detoxification, the effective concentration of the herbicide in the vicinity of the transformed cells is decreased. Such "cross-protection" allows regeneration of untransformed cells, leading to escapes. Christou *et al (Biotechnology,* **9**:957-962 (1991)) observed non-transformed tissue of rice with a regeneration capacity equal to that of transformed tissue, as a result of detoxification of the selective agent by transformed cells.

Finally, Basta marker systems, and indeed Basta resistance as a means of weed control, are used extensively in oilseed rape crops, which are often planted in rotation with wheat crops. Clearly, seed from the previous crop will often be present in the soil and represents the potential for "weed" growth during the next crop cycle. If Basta resistance had been engineered in both the rape and wheat, then use of the herbicide would not be effective against such "weeds".

A further advantage of using a sulphonamide resistance gene as a selectable marker gene in wheat is that the number of insertion sites observed is lower than that observed when the bialaphos resistance is used as the selectable marker. This is advantageous for commercialisation of transgenic plants.

It is desirable to commercialise transgenic plants with only the gene of interest integrated into the plant genome and without any other DNA sequences associated with the transformation procedure. These other sequences include the selectable marker gene required during the transformation process for efficient recovery of transgenic regenerants. The absence of extraneous sequences from the commercial transgenic crop is important for public acceptance. Also, if several herbicide resistance genes are used to produce transgenic wheat, when these are deregulated and used in conventional crossing programmes, the multiply resistant wheat might present a weed problem.

The site of integration of transgenes is random. The selectable marker might be integrated at a site linked to or distant from the gene of interest. It is advantageous to have the selectable marker at few sites as there is then a greater probability of sites being unlinked to the gene of interest and hence segregating away from it at meiosis.

There is therefore a continuing need to provide suitable selectable markers for use in wheat transformation systems. At present there are very few such markers available and this ultimately limits the number of genetic transformations which can be carried out with wheat. That is to say, each time that a new genetic modification is introduced, a separate marker, e.g. a resistance marker, will be required.

EP-A-0369637 described sulphonamide herbicide resistance genes and their utility in both dicot and monocot transformation systems. Particular monocot species listed included wheat. However, the only examples provided related to dicot species and, notwithstanding the comment in that publication concerning use in monocots, the skilled person would, at the filing date of that application, not have known of the problems associated with monocot transformation systems. Given that, as already discussed above, it is now clear that using a dicot marker system in a monocot species can result in, at best, inefficiency of some sort, it is clear that this earlier application was not credible in the context of monocots. Indeed it is clear that, to date, there have not been any reports of the use of a sulphonamide resistance marker system in a monocot species.

Thus, the present invention makes it possible to use a sulphonamide resistance gene as a selectable marker in a wheat species. Suitably, the sulphonamide resistance gene is provided as a DNA construct which encodes a modified dihyropterate synthase (DHPS) eg. having an amino acid sequence as described in EP-B-0369637 (as shown in figure 1 herein) or a sequence modified by one or more amino acid insertions and/or deletions provided that resistance to at least one sulphonamide is conferred on a cell when the gene is expressed therein. In addition, so-called synthetic genes for sulphonamide resistance can be utilised. These can be sequences modified or tailored for particular plant species codon usage, thus ensuring efficient expression.

Preferably, the sulphonamide resistance gene also comprises sufficient regulatory sequences to ensure correct expression, for example a suitable plant promoter. Examples of suitable promoters include the cauliflower mosaic virus 35S promoter, the maize *Adh 1* and *Emu* promoters, the rice actin promoter *Act1* and the maize ubiquitin promoter *Ubi.* The sulphonamide resistance gene may further comprise a sequence encoding a transit peptide cleavable from the modified DHPS fused directly to the 5'-end of the resistance gene. One example of a suitable transit peptide is that for ribulose-1,5-bisphosphate carboxylase/oxygenase.

Thus, the invention makes it possible to use a gene construct comprising:
(a) a plant promoter;
(b) a sulphonamide resistance gene which encodes a modified DHPS; and
(c) a sequence encoding a transit peptide cleavable from the modified DHPS fused directly to the 5' end of the resistance gene;
as a selectable marker in a wheat species.

The present invention provides monocot plant propagating material comprising at least one cell transformed with a DNA construct as defined herein, wherein the propagating material is from wheat.

The present invention provides a transgenic wheat plant, e.g. a wheat plant which contains in its cells a DNA construct as defined herein.

In a further aspect the present invention makes it possible to control weeds at a locus where one or more transgenic plants of the invention are being cultivated which comprises the step of applying to the locus an effective amount of a herbicide which acts by inhibiting DHPS, e.g. Asulam.

In a final aspect the present invention provides a method of producing a wheat plant which is resistant to at least one sulphonamide which comprises:
(a) transforming or transfecting one or more cells derived from a wheat plant with a DNA construct as defined herein;
(b) propagating the transformed or transfected cells from (a) to produce one or more transgenic plants; and
(c) selecting those plants which are resistant to one or more sulphonamide herbicides.

Preferred features of each aspect of the invention are as for each other aspect *mutatis mutandis.*

The invention will now be described with reference to the following examples, which should not be considered as in any way limiting the invention.

Figures are referred to herein in which:

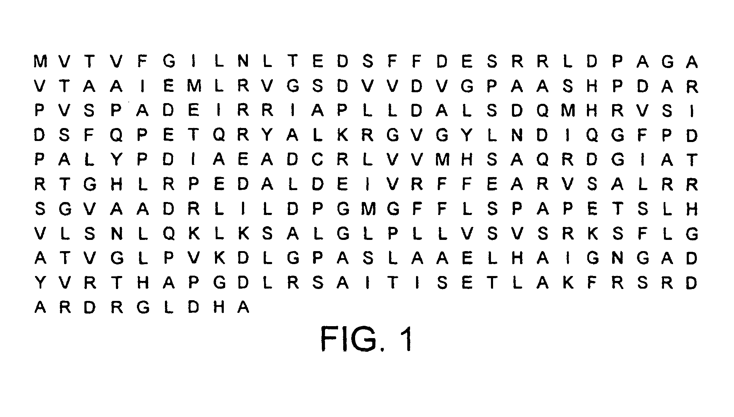

**FIGURE 1:** shows the amino acid sequence of a preferred modified DHPS; and
**FIGURE 2:** shows describes the steps in the construction of plasmid pWP258.

### EXAMPLE 1: Regeneration of transgenic wheat plants using bialaphos resistance as the selectable marker

### Callus induction, maintenance and regeneration

The tissue culture protocol was essentially as the method of Weeks *et al (Plant Physiol.,* **102**:1077-1084 (1993)). Donor plants of the South American spring wheat line designated NB1, obtained from Nickerson Seeds Limited, were grown in a glasshouse under controlled conditions. Immature ears were harvested at 14-20 days post anthesis, and the immature caryopses were removed from the ear and dehusked using forceps. Caryopses were surface-sterilised with 70% ethanol for 2 minutes and 20 % Domestos^{TM} (Lever, UK) for 20-30 minutes and then washed with sterile distilled water. Immature embryos were isolated aseptically under a stereo dissecting microscope and placed scutella uppermost on W3 medium (MS supplemented with 20g/l sucrose and 2mg/l 2,4-D and solidified with 6g/l Type l agarose [Sigma, Poole, UK]). Cultures were maintained at 25°C with a 16 h photoperiod for 5 days prior to bombardment.

Following bombardment, embryos were transferred to selection medium W31B (W3 plus 1mg/l bialaphos [Meija Seika Kaisha Ltd., Yokohama]). Embryos were transferred to fresh W31B medium every 2 weeks. Six weeks after bombardment, all embryos with embryogenic callus were transferred to regeneration medium WR1B (MS supplemented with 20g/l sucrose, and 1mg/l bialaphos and solidified with 6g/l Type I agarose [Sigma, Poole, UK]). Regenerated shoots were transferred to rooting media consisting of ½ strength MS with 1 mg/l bialaphos (1 /2 MS1B). Shoots which produced roots on 1/2MS 1 B were transferred to MS20 (MS medium with 20g/l sucrose).

Plantlets were transferred from MS20 to peat. Samples of leaf tissue were taken from these plantlets for DNA analysis by polymerase chain reaction (PCR). Once established, the plants were transferred to the glasshouse and further leaf tissue was taken for DNA analysis by Southern blot.

### Plasmid DNA

A cotransformation system was used whereby the selectable marker gene and the gene of interest were present on separate plasmids. The *bar* gene from *Streptomyces hygroscopius* conferring resistance to the herbicide bialaphos was carried on the plasmid pDM302 (Cao *et al, Plant Cell Reports,* **11:**586-591 (1992)) under the control of the rice actin 1 (*Act1*) promoter (McElroy *et al*, *Mol. Gen. Genet.,* **231**:150-160 (1991)).

Plasmid DNA was purified from alkaline-lysed cells by CsCl gradients and stored at a concentration of 1 µg/µl in Tris-EDTA buffer, pH 8.0 (Sambrook *et al,* Molecular Cloning: A Laboratory Manual. Cold Spring Harbour Press, Cold Spring Harbour, NY (1989)). A 1:1 mixture of the two plasmids to be cotransformed was prepared immediately prior to coating the microprojectiles.

### Microprojectile Bombardment

Prior to bombardment, tungsten particles (M10; Sylvania, Towanda, PA, USA) were sterilised and coated with plasmid DNA by a procedure adapted from Finer *et al (Plant Cell Reports,* **11**:323-328 (1992)). 50mg of tungsten particles were sterilised in 500µl of 95% ethanol. After 20 minutes the particles were pelleted in a microfuge, washed four times with sterile distilled water then resuspended in 500µl sterile distilled water. 25µl resuspended particles, 7µl plasmid DNA (1 µg/µl), 25µl 2.5 M CaCl₂ and 10µl 100mM spermidine were mixed. After 5 minutes at 4°C, 25µl of supernatant was removed and discarded.

Bombardment was performed using a particle inflow gun (PIG) as described by Finer *et al, (Supra)* whereby DNA-coated particles are accelerated using pressurised helium in combination with a partial vacuum. 2µl of the particle suspension was placed on the screen of the syringe filter unit of the apparatus. Plates of target embryos were placed on a shelf 17cm below the syringe filter unit. A baffle was placed between the syringe filter unit and the target tissue. A vacuum of 100kPa was applied and the particles were discharged when the helium (at 80psi) was released by activation of the soienoid by the timer relay.

### Wheat DNA isolation and analysis

PCR analysis was performed on genomic DNA extracted from 1-2cm² fresh leaf material using a miniprep method described by Stacey and Isaac (Methods in Molecular Biology, Vol. 28: Protocols for nucleic acid analysis by nonradioactive probes, pp9-15, Humana Press Inc., Totawa, NJ (1994)). PCR reactions were performed using primers designed to amplify a 312bp *bar* fragment (5' TGCACCATCGTCAACCACTA 3' and 5' ACAGCGACCACGCTCTTGAA 3'). Reaction conditions were as follows: "hot start" (94°C, 3 min.) followed by 30 cycles of denaturation (95°C, 30 sec.), annealing (55°C, 30 sec.) and extension (65°C, 1 min.) followed by 1 cycle of 75°C (5 min.) and then held at 24°C.

Plants which tested positive by PCR were further analysed by Southern hybridisation. Southern analysis was performed on DNA from a full scale (9ml) extraction from lyophilized ground tissue (Stacey and Isaac, *supra).* DNA samples were adjusted to 0.2mg/ml and digested with the restriction enzyme Xho I. Restriction enzyme digestion, gel electrophoresis and vacuum blotting were carried out as described by Stacey and Isaac (1994, *supra).* A digoxygenin-labelled *bar* probe was produced by PCR according to the method of McCreery and Helentjaris (Methods in Molecular Biology, Vol. 28:Protocols for nucleic acid analysis by non-radioactive probes, pp67-71, Humana Press Inc., Totawa, NJ (1994)). Primers to the 5' region of the CaMV 35S promoter and the 3' end of the *bar* coding region were used to label a 1470bp fragment from pBAR + INT, a pUC based plasmid derived from pJIT84 by inserting the maize *adh1* intron between the 35S promoter and the *bar* gene. Hybridisation of the probe to the Southern blot and detection by chemiluminesence was performed according to the method of McCreery and Helentjaris (Methods in Molecular Biology, Vol. 28:Protocols for nucleic acid analysis by non-radioactive probes, pp 107-112, Humana Press Inc., Totawa, NJ (1994)).

### RESULTS

The number of plant lines containing the *bar* gene were recorded as a percentage of the total number of lines regenerated.

| Experiment No. | No. of Embryos Bombarded | No. of transformed lines produced (as a percentage of the total No. of lines regenerated) |
|---|---|---|
| 1 | 558 | 25% |
| 2 | 821 | 20% |
| 3 | 533 | 100% |
| 4 | 768 | 9% |
| 5 | 849 | 25% |
| 6 | 771 | 50% |
| 7 | 629 | 7% |
| 8 | 656 | 12.5% |
| 9 | 822 | 6% |
| 10 | 864 | 7% |
| 11 | 612 | 6% |
| 12 | 648 | 5% |
| 13 | 797 | . 2% |
| 14 | 668 | 1.5% |
| 15 | 452 | 5% |
| 16 | 523 | 17% |
| 17 | 575 | 5% |
| 18 | 580 | 23.5% |
| 19 | 706 | 8% |
| 20 | 1276 | 10% |

### EXAMPLE 2: ASULAM KILL CURVE

Immature embryos were isolated as described for example 1, plated onto W3 medium and maintained in the dark. After 5-7 days embryos were transferred to W3 medium supplemented with Asulam (Rhone-Poulenc Agrochemicals, Ongar, UK) at a range of concentrations between 0 and 5mg/l and maintained at 25°C with a 16h photo period. Embryos and embryo-derived embryogenic calli were transferred to fresh selective medium every 2 weeks.

The calli were scored for embryogenic response after 6 weeks.

### RESULTS

| Asulam Concentration (mg/l) | Embryogenic response (%)¹ |
|---|---|
| 0 | 100 |
| 0.1 | 100 |
| 0.25 | 64 |
| 0.5 | 28 |
| 1 | 0 |
| 2 | 0 |
| 3 | 0 |
| 4 | 0 |
| 5 | 0 |

The effect of Asulam on the production of embryogenic callus by immature embryos of NB1, ¹% Embryogenic response = (no. embryos with embryogenic callus / total no. embryos plated) x 100.

### EXAMPLE 3: Regeneration of Transgenic wheat plants using Asulam resistance as the selectable marker.

### Callus induction, maintenance and regeneration

Embryos were isolated as described in Example 1 and then maintained at 25°C in the dark for 5 days prior to bombardment. Following bombardment, embryos were cultured at 25°C with a 16h photoperiod, and maintained on W3 medium without selection. One week after bombardment, embryos were transferred to selection medium W32A (W3 plus 2mg/l Asulam). Embryos were transferred to fresh W32A medium every 2 weeks. After 4-6 weeks on selection, all embryos with embryogenic callus were transferred to regeneration medium WR (MS supplemented with 20g/l sucrose, and solidified with 6g/l type l agarose [Sigma, Poole, UK] containing Asulam at a concentration of between 0.1 and 2.0mg/l. After 2 weeks, all healthy tissue was transferred to fresh regeneration medium. After a further 2 weeks any shoots with well developed roots were transferred to MS medium with 20g/l sucrose (MS20).

Plantlets were transferred from MS20 to peat in Magenta GA7 culture vessels (Magenta Corp., Chicago, USA) and allowed to establish before transfer to the glasshouse.

### Plasmid DNA

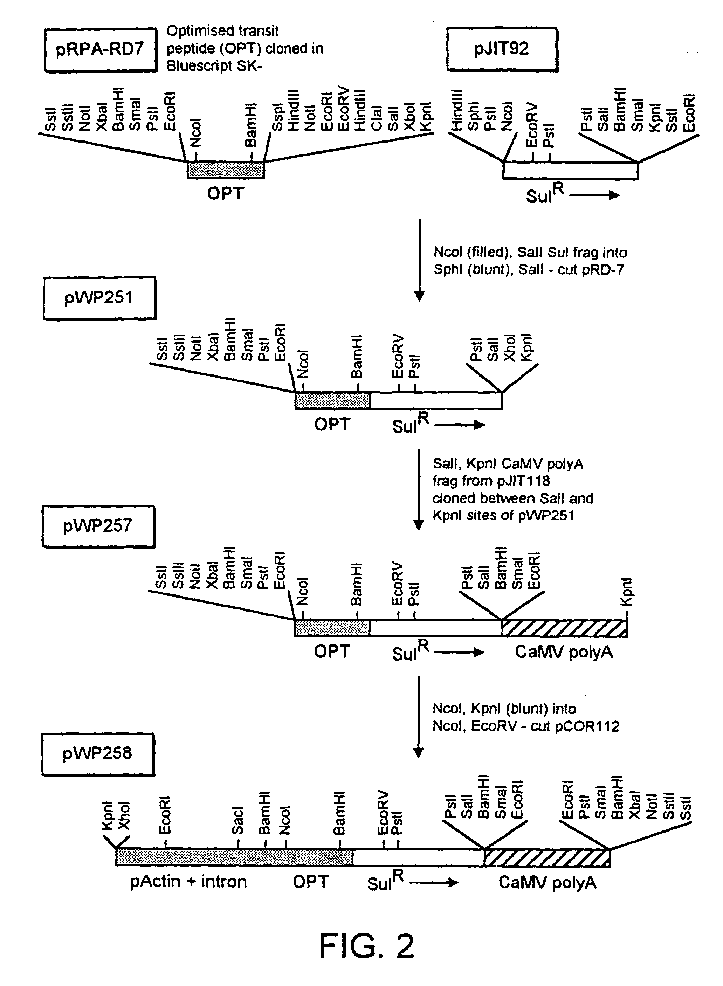

A cotransformation system was used whereby the selectable marker gene and the gene of interest were present on separate plasmids. The sulfonamide resistance trait was carried on the plasmid pWP258 comprising the sul gene from plasmid R46, fused to a chloroplast targeting sequence and under the control of the rice actin 1 *(Act1*) promoter. The plasmid pWP258 was constructed by the following method, which is outlined in Figure 2. The sulfonamide gene was isolated as an Ncol (rendered blunt by filling in the end using T4 DNA polymerase and dNTPs), Sall fragment from pJIT92 (Guerineau *et al, Plant Molecular Biology,* **15:**127-136 (1990)). This fragment was cloned between the Sphl (rendered blunt with T4 DNA polymerase) and the Sall site of pRPA-RD7 (EP-A-0652286, Rhone-Poulenc). The plasmid formed (pWP251) thus encodes the optimized chloroplast transit peptide (OPT) (EP-A-0652286, *supra)* linked in frame to the Sul gene. The CaMV polyadenylation sequence of pJIT118 (Guerineau *et al,* (1990) *supra)* was then cloned between the Sall and Kpnl sites of pWP257. Finally, the OPT-Sul-CaMV PolyA Ncol, Kpnl (rendered blunt with T4 DNA polymerase) fragment of pWP257 was cloned between the Ncol and EcoRV sites of pCOR 112 (McElroy *et al, Mol.Gen.Genet*., **231**:150-160 (1991)) forming pWP258.

Plasmid DNA was purified as described in Example 1.

### Microprojectile bombardment

As for Example 1.

### Wheat DNA isolation and analysis

PCR analysis was performed on genomic DNA extracted as for example 1. PCR reactions were performed using primers designed to amplify a 352bp *sul* fragment (5' TTGTGCGGTTCTTCGAGGC 3' and 5' TGCGCTTCGCAGATCTCCA 3'). Reaction conditions were as follows: hot start (94°C, 3 min.) followed by 30 cycles of denaturation (95°C, 30 sec.), annealing (55°C, 30 sec.) and extension (73°C, 1 min.) followed by 1 cycle of 73°C (5 min.) and then held at 24°C.

### Results

The number of plant lines containing the Sul fragment were recorded as a percentage of the total number of lines regenerated.

| EXPERIMENT No. | No. of embryos Bombarded | No. of transformed lines produced (as a percentage of the total number of lines regenerated) |
|---|---|---|
| 21 | 738 | 100% |
| 22 | 934 | 100% |
| 23 | 720 | 50% |
| 24 | 720 | 100% |
| 25 | 576 | 100% |
| 26 | 648 | 50% |

### EXAMPLE 4: Comparison of number of insertion sites in wheat plants transformed with sul and bar

Southern analysis of *bar* transformed plants was performed as described in Example 1. The number of complete insertions was determined by probing Xho 1 digested genomic DNA (the Xho 1 site provides a unique site digest) and counting the number of resulting bands on the luminograph. Any fragment equal to or larger than the 2210 bp cassette size should contain the whole cassette, anything smaller will only contain partial cassettes.

Southern analysis of *sul* transformed plants was performed according to the method of Example 1, but using a digoxigenin-labelled *sul* probe produced by PCR from pWP258. The number of complete insertions was determined by probing Xho 1 digested genomic DNA (Xho 1 also provides a unique site digest for the pWP258 cassette) and counting the number of resulting bands on the luminograph. Any fragment equal to or larger than the 3500bp cassette size should contain the whole cassette, anything smaller will only contain partial cassettes.

### Results

| Number of insertion sites following *bar* transformation | |
|---|---|
| Line No. | No. of Inserts |
| 1 | 7 |
| 2 | 7 |
| 3 | 3 |
| 4 | 2 |
| 5 | >7 |
| 6 | 2 |
| 7 | 4 |
| 8 | 4/6 |
| 9 | 6 |
| 10 | 5/6 |
| 11 | 5/6 |
| 12 | 1/2 |

| Number of insertion sites following *sul* transformation | |
|---|---|
| Line No. | No. of Inserts |
| 13 | 2 |
| 14 | 5 |
| 15 | 1 |
| 16 | 5 |
| 17 | 2/3 |
| 18 | 2 |
| 19 | 2 |
| 20 | 2 |
| 21 | 2 |
| 22 | 1/2 |

These data suggest that a lower number of insertion sites is achieved by using sulfonamide resistance as the selectable marker than by using bialaphos resistance.

## Claims

1. A monocotyledonous plant propagating material, derived from a wheat species, comprising at least one monocot plant cell transformed or transfected with a DNA construct which encodes a modified dihydropterate synthase (DHPS), wherein expression of said DHPS confers resistance to at least one sulphonamide.

2. The monocotyledonous plant propagating material of claim 1, wherein said DNA construct encodes a modified DHPS having an amino acid sequence as shown in Figure 1, or one substantially homologous thereto.

3. The monocotyledonous plant propagating material of any of claims 1 or 2, wherein said DNA construct further comprises sufficient regulatory sequences to ensure correct expression, e.g. a suitable promoter.

4. The monocotyledonous plant propagating material of any of claims 1 to 3, wherein said DNA construct further comprises a sequence encoding a transit peptide cleavable from the modified DHPS fused directly to the 5' end of the resistance gene.

5. The monocotyledonous plant propagating material of claim 4, wherein said transit peptide is that for ribulose-1, 5-bisphosphate carboxylase/oxygenase.

6. A transgenic wheat plant, comprising at least one cell transformed or transfected with a DNA construct which encodes a modified dihydropterate synthase (DHPS), wherein expression of said DHPS confers resistance to at least one sulphonamide.

7. The transgenic wheat plant of claim 6, wherein said DNA construct encodes a modified DHPS having an amino acid sequence as shown in Figure 1, or one substantially homologous thereto.

8. The transgenic wheat plant of any of claims 6 or 7, wherein said DNA construct further comprises sufficient regulatory sequences to ensure correct expression, e.g. a suitable promoter.

9. The transgenic wheat plant of any of claims 6 to 8, wherein said DNA construct further comprises a sequence encoding a transit peptide cleavable from the modified DHPS fused directly to the 5' end of the resistance gene.

10. The transgenic wheat plant of claim 9, wherein said transit peptide is that for ribulose-1, 5-bisphosphate carboxylase/oxygenase.

11. A seed obtained from a transgenic wheat plant as defined in any of claims 6 to 10, wherein said seed comprises at least one cell transformed or transfected with a DNA construct as defined in any of claims 1 to 5.

12. A method of producing transgenic wheat plants presenting a modified dihydropterate synthase (DHPS) within their genome, comprising the steps of:
(a) transforming or transfecting one or more cells from a wheat plant with a DNA construct as defined in any one of claims 1 to 5;
(b) propagating the transformed or transfected cells from (a) to produce one or more transgenic plants; and
(c) selecting those plants having integrated said DNA construct within their genome.

## Patentansprüche

1. Monokotyledonen-Pflanzenvermehrungsmaterial, das von einer Weizenart stammt und das mindestens eine Monokotyledonen-Pflanzenzelle enthält, die mit einem DNA-Konstrukt, das für eine modifizierte Dihdydropteratsynthase (DHPS) kodiert, transformiert oder transfiziert ist, wobei die Expression dieser DHPS Resistenz gegenüber mindestens einem Sulfonamid verleiht.

2. Monokotyledonen-Pflanzenvermehrungsmaterial nach Anspruch 1, wobei das DNA-Konstrukt für eine modifizierte DHPS mit einer Aminosäuresequenz gemäß Abbildung 1 oder einem ihrer im wesentlichen Homologen kodiert.

3. Monokotyledonen-Pflanzenvermehrungsmaterial nach einem der Ansprüche 1 oder 2, wobei das DNA-Konstrukt weiterhin ausreichende Regulationssequenzen enthält, um eine korrekte Expression zu gewährleisten, z.B. einen geeigneten Promoter.

4. Monokotyledonen-Pflanzenvermehrungsmaterial nach einem der Ansprüche 1 bis 3, wobei das DNA-Konstrukt weiterhin eine Sequenz, die für ein von der modifizierten DHPS abspaltbares Transitpeptid kodiert, in direkter Fusion mit dem 5'-Ende des Resistenzgens umfaßt.

5. Monokotyledonen-Pflanzenvermehrungsmaterial nach Anspruch 4, wobei es sich bei dem Transitpeptid um das Transitpeptid für Ribulose-1,5-bisphosphat-Carboxylase/Oxygenase handelt.

6. Transgene Weizenpflanze, die mindestens eine Zelle umfaßt, die mit einem DNA-Konstrukt, das für eine modifizierte Dihydropteratsynthase (DHPS) kodiert, transformiert oder transfiziert ist, wobei die Expression dieser DHPS Resistenz gegen mindestens ein Sulfonamid vermittelt.

7. Transgene Weizenpflanze nach Anspruch 6, wobei das DNA-Konstrukt für eine modifizierte DHPS mit einer Aminosäuresequenz gemäß Abbildung 1 oder einem ihrer im wesentlichen Homologen kodiert.

8. Transgene Weizenpflanze nach einem der Ansprüche 6 oder 7, wobei das DNA-Konstrukt weiterhin ausreichende Regulationssequenzen enthält, um eine korrekte Expression zu gewährleisten, z.B. einen geeigneten Promoter.

9. Transgene Weizenpflanze nach einem der Ansprüche 6 bis 8, wobei das DNA-Konstrukt weiterhin eine Sequenz, die für ein von der modifizierten DHPS abspaltbares Transitpeptid kodiert, in direkter Fusion mit dem 5'-Ende des Resistenzgens umfaßt.

10. Transgene Weizenpflanze nach Anspruch 9, wobei es sich bei dem Transitpeptid um das Transitpeptid für Ribulose-1,5-bisphosphat-Carboxylase/Oxygenase handelt.

11. Samen von einer transgenen Weizenpflanze nach einem der Ansprüche 6 bis 10, wobei dieser Samen mindestens eine Zelle, die mit einem DNA-Konstrukt, wie in einem der Ansprüche 1 bis 5 definiert ist, transformiert oder transfiziert ist, umfaßt.

12. Verfahren zur Herstellung von transgenen Weizenpflanzen, die innerhalb ihres Genoms eine modifizierte Dihydropteratsynthase (DHPS) aufweisen, das die folgenden Schritte umfaßt:
(a) Transformieren oder Transfizieren einer oder mehrerer Zellen einer Weizenpflanze mit einem DNA-Konstrukt, wie in einem der Ansprüche 1 bis 5 definiert ist;
(b) Vermehren der transformierten oder transfizierten Zellen von (a), wodurch man zu einer oder mehreren transgenen Pflanzen gelangt; sowie
(c) Selektieren derjenigen Pflanzen, die dieses DNA-Konstrukt in ihr Genom integriert haben.

## Revendications

1. Matériel végétal monocotylédone susceptible de se propager, dérivé d'une espèce de blé, comprenant au moins une cellule de plante monocotylée transformée ou transfectée par un construit d'ADN qui code pour une dihydroptérate synthase (DHPS) modifiée, l'expression de ladite DHPS conférant une résistance à au moins un sulfonamide.

2. Matériel végétal monocotylédone susceptible de se propager selon la revendication 1, dans lequel ledit construit d'ADN code pour une DHPS modifiée qui présente la séquence d'acides aminés représentée dans la figure 1 ou une séquence essentiellement homologue de celle-ci.

3. Matériel végétal monocotylédone susceptible de se propager selon l'une quelconque des revendications 1 ou 2, dans lequel ledit construit d'ADN comprend en outre des séquences régulatrices suffisantes pour assurer une expression correcte, par exemple un promoteur approprié.

4. Matériel végétal monocotylédone susceptible de se propager selon l'une quelconque des revendications 1 à 3, dans lequel ledit construit d'ADN comprend en outre une séquence qui code un peptide de transit apte à être scindé de la DHPS modifiée, fusionné directement à l'extrémité 5' du gène de résistance.

5. Matériel végétal monocotylédone susceptible de se propager selon la revendication 4, dans lequel ledit peptide de transit est celui de la ribulose-1,5-biphosphate carboxylase/oxygénase.

6. Blé transgénique qui comprend au moins une cellule transformée ou transfectée par un construit d'ADN qui code une dihydroptérate synthase (DHPS) modifiée, dans lequel l'expression de ladite DHPS confère une résistance à au moins un sulfonamide.

7. Blé transgénique selon la revendication 6, dans lequel ledit construit d'ADN code une DHPS modifiée qui présente la séquence d'acides aminés représentée dans la figure 1 ou une séquence essentiellement homologue de celle-ci.

8. Blé transgénique selon l'une quelconque des revendications 6 ou 7, dans lequel ledit construit d'ADN comprend en outre des séquences régulatrices suffisantes pour garantir une expression correcte, par exemple un promoteur approprié.

9. Blé transgénique selon l'une quelconque des revendications 6 à 8, dans lequel ledit construit d'ADN comprend en outre une séquence qui code un peptide de transit apte à être scindé de la DHPS modifiée, fusionné directement à l'extrémité 5' du gène de résistance.

10. Blé transgénique selon la revendication 9 dans lequel ledit peptide de transit est celui de la ribulose-1,5-biphosphate carboxylase/oxygénase.

11. Semence obtenue à partir d'un blé transgénique selon l'une quelconque des revendications 6 à 10, ladite semence comprenant au moins une cellule transformée ou transfectée par le construit d'ADN tel que défini dans l'une quelconque des revendications 1 à 5.

12. Procédé de production de blés transgéniques qui présentent dans leur génome une dihydroptérate synthase (DHPS) modifiée, ledit procédé comprenant les étapes qui consistent à:
(a) transformer ou transfecter une ou plusieurs cellules d'une plante de blé par le construit d'ADN tel que défini dans l'une quelconque des revendications 1 à 5,
(b) multiplier les cellules transformées ou transfectées de (a) pour produire une ou plusieurs plantes transgéniques et
(c) sélectionner les plantes qui ont intégré ledit construit d'ADN dans leur génome.
